(19) Europäisches Patentamt
European Patent Office
Office européen des brevets

(11) EP 0 844 868 B1

(12) EUROPEAN PATENT SPECIFICATION

(45) Date of publication and mention of the grant of the patent:
**24.10.2001 Bulletin 2001/43**

(21) Application number: **96928418.1**

(22) Date of filing: **06.08.1996**

(51) Int Cl.[7]: **A61K 9/08**, A61K 47/36

(86) International application number:
**PCT/EP96/03477**

(87) International publication number:
**WO 97/06782 (27.02.1997 Gazette 1997/10)**

# (54) Compositions including O-carboxymethyl chitosan and use in ophthalmics

O-Carboxymethylchitosan enthaltende Mittel und ihre Verwendung in Ophthalmika

Composition contenant du O-carboxymethyl-chitosan et utilisation en ophthalmologie

(84) Designated Contracting States:
**AT BE CH DE DK ES FI FR GB GR IE IT LI LU MC NL PT SE**

(30) Priority: **17.08.1995 US 516420**

(43) Date of publication of application:
**03.06.1998 Bulletin 1998/23**

(73) Proprietors:
- **Novartis AG**
  **4056 Basel (CH)**
  Designated Contracting States:
  **BE CH DE DK ES FI FR GB GR IE IT LI LU MC NL PT SE**
- **Novartis-Erfindungen Verwaltungsgesellschaft m.b.H.**
  **1235 Wien (AT)**
  Designated Contracting States:
  **AT**

(72) Inventors:
- **REED, Kenneth, W.**
  **Lawrenceville, GA 30243 (US)**
- **YEN, Shau-Fong**
  **Atlanta, GA 30319 (US)**

(56) References cited:
**EP-A- 0 249 779**
**EP-A- 0 342 557**
**EP-A- 0 426 368**
**EP-A- 0 665 022**
**US-A- 5 422 116**

- **CHEMICAL ABSTRACTS, vol. 113, no. 19, 5 November 1990 Columbus, Ohio, US; abstract no. 165382, BIAGINI G. ET AL: "N-Carboxymethyl chitosan induces neovascularization" XP002019227 & SKJAAK-BRAEK G. ET AL: "Chitin Chitosan : Sources, Chem., Biochem., Phys. Prop., Aplic. ( Proc. Int. Conf. ) 4th" 1989 , ELSEVIER , LONDON**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

This invention relates broadly to compositions including O-carboxyalkyl chitosan and methods of use. More specifically, the invention relates to O-carboxyalkyl chitosan compositions useful in ophthalmic applications.

### DESCRIPTION OF THE RELATED ART

Chitin, poly(N-acetyl-D-glucosamine), is a naturally occurring substance found in shellfish. Chitosan is a partially deacetylated derivative of chitin. More specifically, chitosan is a polysaccharide which consists of N-acetyl-D-glucosamine and D-glucosamine units linked together by $\beta(1\rightarrow4)$ glycosidic bonds. The "degree of deacetylation" in a sample of chitosan refers to the relative amounts of the deacetylated and acetylated monosaccharides present in the chitosan sample. The preparation of chitosan is disclosed in U.S. Patent No. 2,040,880, issued on May 19, 1936.

N,O-carboxyalkyl chitosans are derivatives of chitosan formed by carboxyalkylation of chitosan. The carboxyalkyl groups of N,O-carboxyalkyl chitosan are located at the primary amino group on the D-glycosamine group and at the hydroxyl groups. N,O-carboxymethyl chitosan is water soluble and may be formed by carboxymethylation of chitosan. N,O-carboxymethyl chitosan is commercially available from NOVACHEM, Halifax, N.S., Canada. The preparation of N,O-carboxymethyl chitosan is disclosed in U.S. Patent No. 4,619,995, issued on Oct. 28, 1986, to E. Hayes.

Various uses of chitin and chitosan have been disclosed in the art. For example, P. Sandford, et al., "Biomedical Applications of High Purity Chitosan", Ch. 28, Water-Soluble Polymers, discloses various properties and uses of chitosan.

U.S. Patent No. 4,365,050, issued to Ivani on Dec. 21, 1982, discloses ophthalmic wetting solutions and viscosity builders. These ophthalmic compositions include aminopolysaccharides, primarily N-acetyl-D-glucosamines and derivatives. U.S. Patent No. 4,447,562, issued on May 8, 1984, also to Ivani, discloses pharmaceutical compositions including aminopolysaccharides.

European Patent Application No. 0 426 368 A2, P. Highan, et al., discloses the use of cross-linked biodegradable derivatives of chitin for use in preventing adhesion between body tissues. The preferred chitin derivative is N,O-carboxymethyl-chitosan, but O-carboxy-methyl-chitosan is also suggested as useful in preventing adhesion. In contrast to N,O-carboxyalkyl chitosan, the O-carboxyalkyl derivative of chitosan, has the carboxyalkyl group attached to a free hydroxyl group (typically at the 6 position) of some of the chitosan monosaccharides groups.

EP 665'022 discloses an aqueous viscoelastic solution of N, O-carboxymethyl chitosan for use in ophthalmic surgery.

Chem. Abstr. Vol. 113, 165382 (Biagini et al.) describes the induction of neovascularization when N-carboxymethyl chitosan is inoculated into the cornea. Said cornea resumed its normal functionality within 3 month.

### SUMMARY OF THE INVENTION

An object of the invention is to provide compositions suited for use as excipients in ophthalmic formulations to improve ocular retention and ocular bioavailability.

Another object of the invention is to provide an ophthalmic retention-enhancing material which may be formulated and/or maintained at an acidic pH without excessive turbidity and phase separation.

One embodiment of the invention is an ophthalmic composition including O-carboxymethyl chitosan. In a preferred embodiment, the composition includes a delivery agent which is chemically sensitive to pH, and the pH of the composition is held at an acidic level to enhance stability of the delivery agent.

### BRIEF DESCRIPTION OF THE DRAWINGS

FIG. 1 is a graph of percent miosis v. minutes after instillation for 2% pilocarpine solutions, comparing N,O-carboxymethyl chitosan and hydroxypropylmethyl cellulose.

FIG. 2 is a graph of percent miosis v. minutes after instillation for a 1% pilocarpine solution with hydroxypropylmethyl cellulose and a 0.5% pilocarpine solution with O-carboxymethyl chitosans having three different degrees of carboxymethylation.

FIG. 3 is a graph of percent miosis v. minutes after instillation for 0.5% pilocarpine solutions with chitosan having a 20 minute carboxymethylation time, comparing autoclaved to non-autoclaved samples.

FIG. 4 is a graph of percent miosis v. minutes after instillation for 0.5% pilocarpine solutions with chitosan having

a 180 minute carboxymethylation time, comparing autoclaved to non-autoclaved samples.

DESCRIPTION OF THE PREFERRED EMBODIMENTS

One embodiment of the present invention is an O-carboxymethyl chitosan-containing composition for use in ophthalmic products. Ophthalmic products include a wide range of products intended for intimate contact with the ocular environment, i.e., eye tissue, ocular surrounding fluids, or tissue surrounding the eye. O-carboxymethyl chitosan may be used to improve ocular retention and ocular bioavailability in ophthalmic solutions. Further, O-carboxymethyl chitosan may be used in enteric-coated agent delivery devices, which are those devices which provide some control in the delivery of an agent to the gastrointestinal tract. The term "agent", as used herein, means drug, pharmaceutical, diagostic agent, vitamin, or other agent which is advantageous to delivery to the ocular environment.
O-carboxymethyl chitosan, as used herein, is defined as follows:

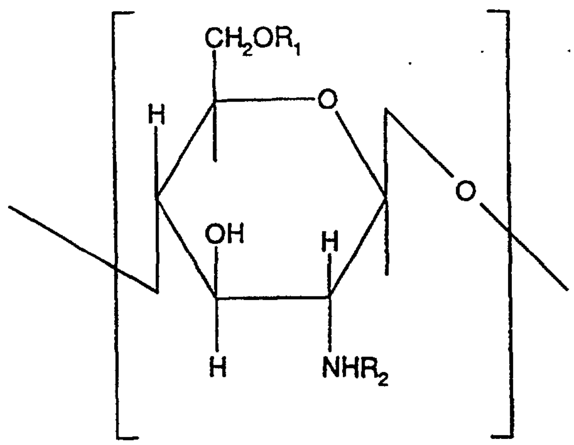

where $R_1$ is H 0 to 82% of the time and carboxymethyl 18-100% of the time, and where $R_2$ is H 50 to 100% of the time and acetyl 0 to 50% of the time. The O at the 3-position may be substituted with $R_1$ some of the time, but the species shown is the predominate species.

OPHTHALMIC COMPOSITIONS AND METHODS

It has been discovered that O-carboxymethyl chitosan has certain properties which may be advantageously used in ophthalmic products or in enteric coatings. One advantage of O-carboxymethyl chitosan relates to retention-enhancing or bioavailability-enhancing characteristics, while the other relates to the pH of the formulation. First, it has been discovered that O-carboxymethyl chitosan provides better bioavailability of delivery agents than does N,O-carboxymethyl chitosan. Thus, O-carboxymethyl chitosan compositions have demonstrated unexpected advantages over N,O-carboxymethyl chitosan compositions in enhancing ocular retention or bioavaibility of ophthalmic delivery agents.
A second advantage of O-carboxymethyl chitosan compositions relates to the ability to maintain and/or formulate a delivery composition at an acidic or neutral pH. Chitosan precipitates out of solution when adjusted to a physiological pH of about 7.4, which is the pH of human tear fluid. Precipitation of an ophthalmic retention-enhancing agent upon contact with the eye may cause blurred vision. In contrast to chitosan, O-carboxymethyl chitosan, which has water soluble carboxymethyl groups substituted at the O-position, has been found to remain soluble at the pH of the tear fluid. Similarly, N,O-carboxymethyl chitosan is also soluble in tear (ocular) fluid at physiological pH. Thus, neither O-carboxymethyl chitosan nor N,O-carboxymethyl chitosan will precipitate out when applied to the eye.
However, N,O-carboxymethyl chitosan cannot be formulated at a pH much lower than physiological pH (i.e., pH = 7.4). Turbidity and phase separation occurs in N,O-carboxymethyl chitosan-containing compositions at a pH of about 6.6 or lower. Although a physiological pH of 7.4 is preferred for application to the eye in order to maximize patient comfort, many active agents require a lower pH in order to avoid stability problems (i.e., to enhance shelf life). Thus, while it is commonly accepted that a pH of 6 to 8 is comfortable to the eye, stability problems require that some commercial formulations maintain pH values as low as 3. For example, pilocarpine formulations are typically held at a pH of 5 or lower.
While compositions may be formulated with N,O-carboxymethyl chitosan at a pH lower than 6.6, the phase separation requires the consumer to agitate the composition before use. Also, immediately subsequent to application

to the eye, the consumer will experience blurred vision because of the turbidity. Further, turbid ophthalmic compositions are not aesthetically appealing to the consumer.

In contrast, O-carboxymethyl chitosan may be formulated at a pH of 4 or higher without precipitation out of solution. Thus, preferably, the ophthalmic formulation has a pH of 4 or higher, more preferably 4.5 or higher. In a preferred embodiment, the ophthalmic solution has a pH of 4 to 9. In another preferred embodiment, the ophthalmic solution has a pH of 4 to 6. O-carboxymethyl chitosan is commercially available from Nova Chem., Ltd., Halifax, N.S., Canada.

The O-carboxymethyl chitosan molecular weight may range from about 1000 Daltons to about 5,000,000 Daltons, depending on the intended use of the final product. The compositions of the present invention may have a viscosity of about 1 to 200,000 mPa·s (centipoise) at 25°C, depending again on the intended use of the product. A preferred viscosity range for an ophthalmic product which delivers an agent to the ocular environment is 50 to 100,000 mPa·s (centipoise.) A preferred viscosity range for an artificial tears product is 50 to 500 mPa·s (centipoise.)

The present O-carboxymethyl chitosan ophthalmic compositions are especially advantageous in delivery of agents to the ocular environment, i.e., to the tear fluid, eye, or surrounding ocular tissues. A wide variety of agents may be delivered in accordance with the present invention, including, without limitation, beneficial pharmaceutical agents, diagnostic agents, vitamins, nutrients, lubricants, and the like. The ophthalmic delivery agent may include, without limitation thereto, 3H-thymidine, acetylcholine chloride, acyclovir, adrenaline, amethocaine, aminocaproic acid, antazoline phosphate, arachidonic acid, atropine, benoxinate hydrochloride, betaxolol hydrochloride, bupivacaine, carbachol, carteolol, chloramphenicol, chlortetracycline hydrochloride, chymatrypsin, clonidine, cocaine, corynanthine, cromolyn sodium, cyclopentolate, demecarium bromide, dexamethasone, dibutoline, dichlorphenamide, diclofenac, dipivefrin hydrochloride, echodtiophate iodide, ephedrine, epinephrine bitartrate, erythromycin, ethambutol, etidocaine, eucatropine, fluoromethalone, fluorometholone, gentamicin sulfate, gramicidine, H-thymidine, homatropine hydrobromide, hyaluronic acid, hydrocortisone, idoxuridine, indomethacin, inositol triphosphate, inositol phosphates, isoflurophate, isosorbide, lachesine, levobunolol, levocabastine, lidocaine, lignocaine, medrysone, mepivacaine, methacholine, methazolamide, naphazoline hydrochloride, natamycin, neomycin sulfate, neostigmine, noradrenaline, ofloxacin, oxybuprocaine, oxymetazolin, oxyphenonium, pheniramine maleate, phenylephrine hydrochloride, phosphatidylinositol phosphates, physostigmine, pilocarpine hydrochloride, polyhexamethylene biguanides, polymyxin B sulfates, prednisolone sodium phosphate, proparacaine hydrochloride, proxymethacaine, pyrilamine maleate, scopolamine hydrobromide, sorbinil, sulfacetamide, sulfisoxazole disolamine, tamoxifen, tetracaine hydrochloride, tetracycline, tetrahydrozoline hydrochloride, timolol maleate and hemihydrate, trifluridine, tropicamide, vidarabine, and other ophthalmically acceptable salts thereof and mixtures thereof.

One preferred set of delivery agents includes those which degrade during storage in solutions at a pH which is not acidic, i.e., "pH-sensitive delivery agents". Thus, one preferred group of ophthalmic delivery agents includes pilocarpine, epinephrine, dipivefrin, hydralazine, carbachol, ophthalmically acceptable salts thereof and mixtures thereof. Particularly preferred delivery agents include hydralazine and pilocarpine, and ophthalmically accceptable salts thereof.

The ophthalmic compositions of the present invention include 0.1 to 25 weight percent O-carboxymethyl chitosan. More preferably, the ophthalmic product includes about 1 to about 10 weight percent O-carboxymethyl chitosan.

In a preferred embodiment, the ophthalmic compositions include (a) 0.01 to 10 weight percent of a delivery agent; (b) 0.1 to 25 weight percent O-carboxymethyl chitosan; and (c) 99.89 to 74.99 weight percent ophthalmic carrier. A more preferred ophthalmic composition includes (a) 0.01 to 2.0 weight percent of a delivery agent; (b) 1 to 6 weight percent O-carboxymethyl chitosan; and (c) 98.99 to 93.99 weight percent ophthalmic carrier.

Ophthalmic carrriers may be chosen from a wide variety of carriers known in the art which are ophthalmically acceptable. Ophthalmic carriers include, without limitation thereto, water, petrolatum, mineral oil, silicone oil, and natural vegetable oils such as olive oil.

In a preferred embodiment, the O-carboxymethyl chitosan (or composition containing O-carboxymethyl chitosan) is subjected to autoclaving. Autoclaving at elevated temperatures is useful for sterilizing ophthalmic compositions. However, a disadvantage of autoclaving, for some ophthalmic compositions, is that the ocular retention-enhancing component may be degraded, decomposed, or otherwise damaged. Damage to the retention-enhancing component (s) ultimately reduces the bioavailability of the delivery agent upon application to the eye. However, it has been surprisingly found that autoclaving O-carboxymethyl chitosan can improve the ocular retention-enhancing characteristics. This increase in retentionenhancing characteristics is more pronounced with higher levels of carboxymethylation at the O-position. The temperature and duration of autoclaving may vary depending on the specific composition and application, but a temperature of 100 to 150°C for a period of 5 to 60 minutes is a useful range. Thus, in a preferred embodiment, the O-carboxymethyl chitosan, or composition thereof, is autoclaved at elevated temperatures.

The O-carboxyalkyl chitosan-containing ophthalmic compositions which are prepared at an acidic pH for storage may be altered to increase the pH immediately prior to administration of the solution to the ocular environment. It is generally accepted that a pH of 6 to 8 does not produce patient discomfort, i.e., a pH of 6 to 8 is ocularly compatible.

There are numerous methods of increasing the pH of the ophthalmic solution immediately prior to contact with the eye. For example, U.S. Patent Nos. 5,056,689 and 5,080,800, issued to Heyl, et al., disclose ophthalmic dispensers including scavenger media positioned between the solution and the dispenser outlet. In operation, the media removes a component of the solution when the consumer passes the solution through the media while dispensing the solution to the eye. In the case of low pH solutions, a media which increases the solution pH upon contact may be provided in the dispenser tip. Accordingly, a particularly suitable scavenger media is preferably selected from a negatively and/or a positively charged scavenging material, e.g. an ion exchange resin. A particular example is a scavenging material comprised of a mixture of "Bio Rex 5" and "AG-4", both Bio Rad products, in a 75 to 25 ratio, which will almost completely remove 0.1% sorbic acid from an aqueous solution and raise the pH of the solution from 4.0 to about 7.0.

The previous disclosure will enable one having ordinary skill in the art to practice the invention. In order to better enable the reader to understand specific embodiments and the advantages thereof, reference to the following examples is suggested.

EXAMPLE I

An aqueous solution containing 2 weight percent pilocarpine and four (4) weight percent N,O-carboxymethyl chitosan is prepared as follows. 5 grams glacial acetic acid and about 6 grams sodium chloride is added to about 900 ml of deionized water. 40 grams of N,O-carboxymethyl chitosan (Protan Laboratories, Redmond, VA) is dissolved in the aqueous solution. 20 grams of pilocarpine (Sigma Chemical Co.) is added to the solution, with the pH being adjusted to about 5 by adding 1 N HCl. The final volume is adjusted q.s. to 1 liter.

The resultant solution is evaluated by measuring the pupil diamter of rabbit eyes at various times after instillation of 30 microliters of the solution into the rabbit eye, and calculating miotic effect from the measured pupil diameters. Miosis is a relative measure of pupil constriction. Administration of pilocarpine to the eye causes the pupil to constrict. Thus, the effectiveness of a pilocarpine delivery solution may be expressed by "percent miosis". "Percent miosis", as used herein, is defined by the following equation:

$$\text{percent miosis} = (D_{new}-D_{base})/D_{base}\cdot 100$$

where

$D_{base}$ = baseline pupil diameter (prior to solution contact)
$D_{new}$ = new pupil diameter after a given contact time with the test solution

Averaged percent miosis as a function of time from instillation into the eye is presented in TABLE 1.

EXAMPLE II

An aqueous solution containing 2 weight percent pilocarpine and 4.5 mg/ml hydroxypropyl methylcellulose (HPMC) is evaluated. The solution is a SPERSACARPINE™ solution, which is commercially available from CIBA Vision, AG (Dispersa, AG), Hettlingen, Switzerland.

TABLE 1 gives the averaged percent miosis as a function of time from instillation into the eye. FIG.1 graphically illustrates the data of TABLE 1.

TABLE 1

| Time (minutes) from instillation in rabbit eye | Percent Miosis for 2% pilocarpine with 4.5 mg/ml HPMC (SPERSACARPINE™) | Percent Miosis for 2% pilocarpine in 4% N,O-carboxymethyl chitosan |
|---|---|---|
| 0 | 0.0 | 0.0 |
| 20 | 47.0 | 40.7 |
| 30 | 44.9 | 36.4 |
| 40 | 41.0 | 29.1 |
| 60 | 32.4 | 26.5 |

TABLE 1 (continued)

| Time (minutes) from instillation in rabbit eye | Percent Miosis for 2% pilocarpine with 4.5 mg/ml HPMC (SPERSACARPINE™) | Percent Miosis for 2% pilocarpine in 4% N,O-carboxymethyl chitosan |
|---|---|---|
| 120 | 25.0 | 14.5 |
| 180 | 10.4 | 8.7 |
| 240 | 6.0 | 1.8 |

EXAMPLES I and II and FIG. 1 illustrate that 4.5 mg/ml hydroxypropylmethyl cellulose performs similarly to 4 weight percent N,O-carboxylmethyl chitosan in miosis profile as a function of time. However, HPMC performs slightly better than N,O-carboxymethyl chitosan with pilocarpine, as indicated by magnitude of miosis at a given time.

EXAMPLE III

The procedures outlined in EXAMPLE I are used to test a SPERSACARPINE™ solution having one (1) weight percent pilocarpine and 4.5 mg/ml HPMC. The data is presented in TABLE 2 and FIG. 2.

EXAMPLE IV

3 grams of 180-minute carboxymethylated O-carboxymethyl chitosan (Nova Chem., Ltd.) is dissolved in about 80 ml of distilled water. pH is adjusted to 12 with sodium hydroxide. About 1.6 grams mannitol is added to adjust osmolality to near isotonic. The solution is autoclaved in a Yamato SM32 autoclave at 121°C for 15 minutes. The pH is adjusted to 5 with concentrated HCl. 0.5 grams pilocarpine hydrochloride is added to the solution. The volume is adjusted q.s. to 100 ml.

The resulting solution, including 0.5 weight percent pilocarpine in three (3) weight percent of 180-minute-carboxymethylated O-carboxymethyl chitosan, is evaluated as outlined in EXAMPLE I. The data is presented in TABLE 2 and FIG. 2.

EXAMPLE V

1.5 grams of 20-minute carboxymethylated O-carboxymethyl chitosan (Nova Chem., Ltd.) is dissolved in 40 ml of distilled water. The pH is adjusted to 12 with sodium hydroxide. 0.9 grams mannitol is added to adjust the osmolality to near isotonic. The solution is autoclaved in a Yamato SM32 autoclave at 121°C for 15 minutes. The pH is adjusted to 5 with concentrated HCl. 0.25 grams pilocarpine hydrochloride is added to the solution. The volume is adjusted q. s. to 50 ml.

The resulting solution, including 0.5 weight percent pilocarpine in three (3) weight percent of 20-minute-carboxymethylated O-carboxymethyl chitosan, is evaluated as outlined in EXAMPLE I. The data is presented in TABLE 2 and FIG. 2.

EXAMPLE VI

1.5 grams of 60-minute carboxymethylated O-carboxymethyl chitosan (Nova Chem., Ltd.) is dissolved in 40 ml of distilled water. The pH is adjusted to 12 with sodium hydroxide. 0.8 grams mannitol is added to adjust osmolality to near isotonic. The solution is autoclaved in a Yamato SM32 autoclave at. 121°C for 15 minutes. The pH is adjusted to 5 with concentrated HCl. 0.25 grams pilocarpine hydrochloride is added to the solution. The volume is adjusted q. s. to 50 ml.

The resulting solution, 0.5 weight percent pilocarpine in three (3) weight percent of 60-minute-carboxymethylated O-carboxymethyl chitosan, is evaluated as outlined in EXAMPLE I. The data is presented in TABLE 2 and FIG. 2.

TABLE 2

| | Percent miosis | | | |
|---|---|---|---|---|
| | | Autoclaved | | |
| Time (minutes) from instillation in rabbit eye | 1% pilocarpine with 4.5 mg/ml HPMC (SPERSACARPINE™) | 0.5% pilocarpine in 3% of 20-min.-CM O-CM chitosan | 0.5% pilocarpine in 3% of 60-min.-CM O-CM chitosan | 0.5% pilocarpine in 3% of 180-min.-CM O-CM chitosan |
| 0 | 0.0 | 0.0 | 0.0 | 0.0 |
| 20 | 32.8 | | | |
| 25 | | 27.7 | 37.1 | 45.1 |
| 30 | 27.3 | | | |
| 40 | 21.8 | 30.7 | 34.6 | 43.8 |
| 60 | 16.3 | 24.4 | 32.1 | 42.9 |
| 120 | 8.0 | 17.2 | 23.3 | 33.8 |
| 180 | 3.7 | 7.6 | 13.3 | 31.1 |
| 240 | 0.0 | 0.0 | 3.3 | 26.0 |

EXAMPLES III-VI and FIG. 2 illustrate that a lower pilocarpine concentration (0.5%) in O-carboxylmethyl chitosan performs more effectively than a higher pilocarpine concentration (1%) in 4.5 mg/ml hydroxypropylmethyl cellulose. Thus, O-carboxymethyl chitosan performs more effectively as an ophthalmic drug delivery vehicle than HPMC or N,O-carboxymethyl chitosan. EXAMPLES IV-VI and FIG. 2 also illustrate that the effectiveness of O-carboxylmethyl chitosan increases with time of carboxymethylation.

EXAMPLE VII

30 grams of O-carboxymethyl chitosan (Nova Chem. Limited, Nova Scotia, Canada) having a 180 minute carboxymethylation time is dissolved in 900 ml of deionized water. 30 grams of mannitol, a tonicity agent, and 5 grams pilocarpine are added to the solution. The pH is adjusted to 4.7 by adding 37% HCl solution. The final volume is adjusted to one liter with deionized water. No autoclaving is performed on the O-carboxymethyl chitosan. Data is presented in TABLE 3. A graphical comparison of autoclaved (EXAMPLE IV) and non-autoclaved (EXAMPLE VII) at 180-minutes carboxymethylation delivery solutions is presented in FIG. 3.

EXAMPLE VIII

An aqueous O-carboxymethyl chitosan/pilocarpine solution is prepared as in Example VII, with the exception being that a 20-minute-carboxymethylated chitosan is used. Data is presented in TABLE 3. A graphical comparison of autoclaved (EXAMPLE V) and non-autoclaved (EXAMPLE VIII) at 20 minutes carboxymethylation delivery solutions is presented in FIG. 4.

TABLE 3

| Time (minutes) from instillation in eye | 0.5% pilocarpine in 3% of **20-min-** carboxymethylated O-carboxymethyl chitosan **- no autoclaving -** | 0.5% pilocarpine in 3% of **180-min-** carboxymethylated O-carboxymethyl chitosan **- no autoclaving -** |
|---|---|---|
| 0 | 0.0 | 0.0 |
| 25 | 27.8 | 34.5 |
| 40 | 34.0 | 38.8 |
| 60 | 32.0 | 31.2 |
| 120 | 23.7 | 22.2 |

TABLE 3 (continued)

| Time (minutes) from instillation in eye | 0.5% pilocarpine in 3% of **20-min-** carboxymethylated O-carboxymethyl chitosan **- no autoclaving -** | 0.5% pilocarpine in 3% of **180-min-** carboxymethylated O-carboxymethyl chitosan **- no autoclaving -** |
|---|---|---|
| 180 | 13.4 | 11.7 |
| 240 | 3.1 | 4.2 |

A comparison of EXAMPLES IV and V with EXAMPLES VII and VIII, respectively, and an examination of FIGS. 3 and 4 reveal that autoclaving improves the effectiveness of O-carboxymethyl chitosan for highly carboxymethylated chitosan (180-min. carboxymethylation period) but reduces effectiveness for low carboxymethylated chitosan (20-minutes carboxymethylation period).

**Claims**

**1.** An ophthalmic composition, comprising:

(a) 0.1 to 25 weight percent O-carboxymethyl chitosan of formula (I),

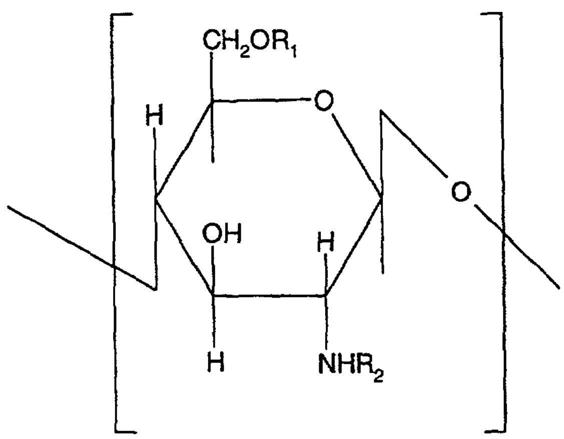

(I)

wherein

$R_1$ is H 0% to 82% of the time and carboxymethyl 18%-100% of the time, and where $R_2$ is H 50% to 100% of the time and acetyl 0% to 50% of the time; and wherein
the O at the 3-position may be substituted with $R_1$ some of the time, but the species shown is the predominate species,

(b) 0.01 to 10 weight percent of an ophthalmic delivery agent; and
(c) 99.89 to 74.99 weight percent ophthalmic carrier.

**2.** An ophthalmic composition of claim 1, wherein said ophthalmic composition has a pH of 4 or higher.

**3.** An ophthalmic composition of claim 1, comprising 1 to 10 weight percent O-carboxymethyl chitosan.

**4.** An ophthalmic composition of claim 1, comprising:

(a) 1 to 6 weight percent O-carboxymethyl chitosan;
(b) 0.01 to 2 weight percent of an ophthalmic delivery agent; and
(c) 98.99 to 93.99 weight percent ophthalmic carrier.

5. An ophthalmic composition of claim 1, wherein said delivery agent degrades during storage in solutions at a pH, which is not acidic.

6. An ophthalmic composition of claim 1, wherein said delivery agent is selected from the group consisting of 3H-thymidine, acetylcholine chloride, acyclovir, adrenaline, amethocaine, aminocaproic acid, antazoline phosphate, arachidonic acid, atropine, benoxinate hydrochloride, betaxolol hydrochloride, bupivacaine, carbachol, carteolol, chloramphenicol, chlortetracycline hydrochloride, chymatrypsin, clonidine, cocaine, corynanthine, cromolyn sodium, cyclopentolate, demecarium bromide, dexamethasone, dibutoline, dichlorphenamide, diclofenac, dipivefrin hydrochloride, echodtiophate iodide, ephedrine, epinephrine bitartrate, erythromycin, ethambutol, etidocaine, eucatropine, fluoromethalone, fluorometholone, gentamicin sulfate, gramicidine, H-thymidine, homatropine hydrobromide, hyaluronic acid, hydrocortisone, idoxuridine, indomethacin, inositol triphosphate, inositol phosphates, isoflurophate, isosorbide, lachesine, levobunolol, levocabastine, lidocaine, lignocaine, medrysone, mepivacaine, methacholine, methazolamide, naphazoline hydrochloride, natamycin, neomycin sulfate, neostigmine, noradrenaline, ofloxacin, oxybuprocaine, oxymetazolin, oxyphenonium, pheniramine maleate, phenylephrine hydrochloride, phosphatidylinositol phosphates, physostigmine, pilocarpine hydrochloride, polyhexamethylene biguanides, polymyxin B sulfates, prednisolone sodium phosphate, proparacaine hydrochloride, proxymethacaine, pyrilamine maleate, scopolamine hydrobromide, sorbinil, sulfacetamide, sulfisoxazole disolamine, tamoxifen, tetracaine hydrochloride, tetracycline, tetrahydrozoline hydrochloride, timolol maleate and hemihydrate, trifluridine, tropicamide, vidarabine, and other ophthalmically acceptable salts thereof and mixtures thereof.

7. An ophthalmic composition of claim 1, wherein said delivery agent is selected from the group consisting of pilocarpine, epinephrine, dipivefrin, hydralazine, carbachol, ophthalmically acceptable salts thereof and mixtures thereof.

8. An ophthalmic composition of claim 7, wherein said delivery agent is hydralazine or an ophthalmically acceptable salt thereof.

9. An ophthalmic composition of claim 1, wherein said composition has a viscosity of 50 to 500 mPa s (centipoises) at 25°C.

10. An ophthalmic composition of claim 1, wherein said composition is an artificial tears fluid.

11. An ophthalmic composition of claim 1, wherein said composition has a viscosity of 50 to 100,000 mPa s (centipoises) at 25°C.

12. An ophthalmic composition of claim 1, comprising 1 to 6 weight percent O-carboxymethyl chitosan.

13. An ophthalmic composition of claim 1, comprising:

(a) 1 to 6 weight percent O-carboxymethyl chitosan;
(b) 0.01 to 2 weight percent of an ophthalmic delivery agent; and
(c) 98.99 to 93.99 weight percent ophthalmic carrier.

14. An ophthalmic composition of claim 14, wherein said delivery agent is selected from the group consisting of pilocarpine, epinephrine, dipivefrin, hydralazine, carbachol, ophthalmically acceptable salts thereof and mixtures thereof; and wherein the pH of said ophthalmic composition is above 4.

15. An ophthalmic composition of claim 1, wherein said O-carboxymethyl chitosan has been subjected to autoclaving at elevated temperatures of 100 to 150°C for a time of 5 to 60 minutes.

16. Use of O-carboxymethyl chitosan of formula (I) of claim 1 in the preparation of an ophthalmic composition of claim 1 to enhance ocular retention and/or bioavailability of an ophthalmic delivery agent.

## Patentansprüche

1. Ophthalmische Zusammensetzung, die die folgenden Bestandteile umfaßt:

(a) 0,1 bis 25 Gew.-% O-Carboxymethylchitosan der Formel (I):

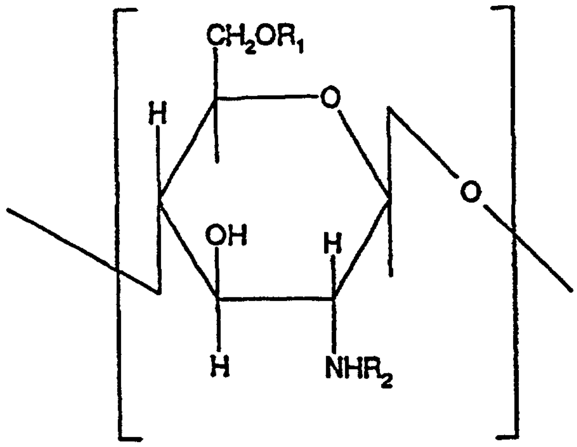

(I)

worin $R_1$ in 0-82% der Fälle für H und in 18-100% der Fälle für Carboxymethyl steht und $R_2$ in 50-100% der Fälle für H und 0-50% der Fälle für Acetyl steht, und wobei das Sauerstoffatom in 3-Stellung in einigen Fällen durch $R_1$ substituiert sein kann, wobei jedoch die dargestellte Spezies die vorwiegende Spezies ist,
(b) 0,01 bis 10 Gew.-% eines ophthalmischen Abgabemittels und
(c) 99,89 bis 74,99 Gew.-% eines ophthalmischen Trägers.

**2.** Ophthalmische Zusammensetzung nach Anspruch 1, wobei die ophthalmische Zusammensetzung einen pH-Wert von 4 oder mehr aufweist.

**3.** Ophthalmische Zusammensetzung nach Anspruch 1, die 1 bis 10 Gew.-% O-Carboxymethylchitosan umfaßt.

**4.** Ophthalmische Zusammensetzung nach Anspruch 1, die

(a) 1 bis 6 Gew.-% O-Carboxymethylchitosan,
(b) 0,01 bis 2 Gew.-% eines ophthalmischen Abgabemittels und
(c) 98,99 bis 93,99 Gew.-% eines ophthalmischen Trägers umfaßt.

**5.** Ophthalmische Zusammensetzung nach Anspruch 1, wobei das Abgabemittel während der Lagerung in Lösungen bei einem pH-Wert, der nicht sauer ist, abgebaut wird.

**6.** Ophthalmische Zusammensetzung nach Anspruch 1, wobei das Abgabemittel aus 3H-Thymidin, Acetylcholinchlorid, Acyclovir, Adrenalin, Amethocain, Aminocapronsäure, Antazolinphosphat, Arachidonsäure, Atropin, Benoxinathydrochlorid, Betaxololhydrochlorid, Bupivacain, Carbachol, Carteolol, Chloramphenicol, Chlortetracyclinhydrochlorid, Chymatrypsin, Clonidin, Cocain, Corynanthin, Cromolynnatrium, Cyclopentolat, Demecariumbromid, Dexamethason, Dibutolin, Dichlorphenamid, Diclofenac, Dipivefrinhydrochlorid, Echodtiophatiodid, Ephedrin, Epinephrinbitartrat, Erythromycin, Ethambutol, Etidocain, Eucatropin, Fluormethalon, Fluormetholon, Gentamycinsulfat, Gramicidin, H-Thymidin, Homatropinhydrobromid, Hyaluronsäure, Hydrocortison, Idoxuridin, Indomethacin, Inositoltriphosphat, Inositolphosphate, Isoflurophat, Isosorbid, Lachesin, Levobunolol, Levocabastin, Lidocain, Lignocain, Medryson, Mepivacain, Methacholin, Methazolamid, Naphazolinhydrochlorid, Natamycin, Neomycinsulfat, Neostigmin, Noradrenalin, Ofloxacin, Oxybuprocain, Oxymetazolin, Oxyphenonium. Pheniraminemaleat, Phenylephrinhydrochlorid, Phosphatidylinositolphosphate, Physostigmin, Pilocarpinhydrochorid, Polyhexamethylenbiguanide, Polymyxin-B-sulfate, Prednisolonnatriumphosphat, Proparacainhydrochlorid, Proxymethacain, Pyrilaminmaleat, Scopolaminhydrobromid, Sorbinil, Sulfacetamid, Sulfisoxazoldisolamin, Tamoxifen, Tetracainhydrochlorid, Tetracyclin, Tetrahydrozolinhydrochlorid, Timololmaleat und -hemihydrat, Trifluridin, Tropicamid, Vidarabin und andere ophthalmisch akzeptable Salze hiervon und Gemische ausgewählt ist.

**7.** Ophthalmische Zusammensetzung nach Anspruch 1, wobei das Abgabemittel aus Pilocarpin, Epinephrin, Dipivefrin, Hydralazin, Carbachol, ophthalmisch akzeptablen Salzen hiervon und Gemischen hiervon ausgewählt ist.

**8.** Ophthalmische Zusammensetzung nach Anspruch 7, wobei das Abgabemittel Hydralazin oder ein ophthalmisch akzeptables Salz hiervon ist.

**9.** Ophthalmische Zusammensetzung nach Anspruch 1, wobei die Zusammensetzung eine Viskosität von 50 bis 500 mPa·s (Centipoise) bei 25°C aufweist.

**10.** Ophthalmische Zusammensetzung nach Anspruch 1, wobei die Zusammensetzung eine künstliche Tränenflüssigkeit ist.

**11.** Ophthalmische Zusammensetzung nach Anspruch 1, wobei die Zusammensetzung eine Viskosität von 50 bis 100.000 mPa·s (Centipoise) bei 25°C aufweist.

**12.** Ophthalmische Zusammensetzung nach Anspruch 1, die 1 bis 6 Gew.-% O-Carboxymethylchitosan umfaßt.

**13.** Ophthalmische Zusammensetzung nach Anspruch 1, die

(a) 1 bis 6 Gew.-% O-Carboxymethylchitosan,
(b) 0,01 bis 2 Gew.-% eines ophthalmischen Abgabemittels und
(c) 98,99 bis 93,99 Gew.-% eines ophthalmischen Trägers umfaßt.

**14.** Ophthalmische Zusammensetzung nach Anspruch 13, wobei das Abgabemittel aus Pilocarpin, Epinephrin, Dipivefrin, Hydralazin, Carbachol, ophthalmisch akzeptablen Salzen und Gemischen hiervon ausgewählt ist und wobei der pH-Wert der ophthalmischen Zusammensetzung größer 4 ist.

**15.** Ophthalmische Zusammensetzung nach Anspruch 1, wobei das O-Carboxymethylchitosan einem Autoklavieren bei erhöhter Temperatur von 100-150°C während einer Zeitdauer von 5-60 min unterzogen wurde.

**16.** Verwendung eines O-Carboxymethylchitosans der Formel (I) nach Anspruch 1 bei der Herstellung einer ophthalmischen Zusammensetzung nach Anspruch 1 zur Verbesserung der Augenretention und/oder Bioverfügbarkeit eines ophthalmischen Abgabemittels.

## Revendications

**1.** Une composition ophtalmique comprenant :

(a) 0,1 à 25 pourcents en poids d'O-carboxyméthyl-chitosan de formule (I),

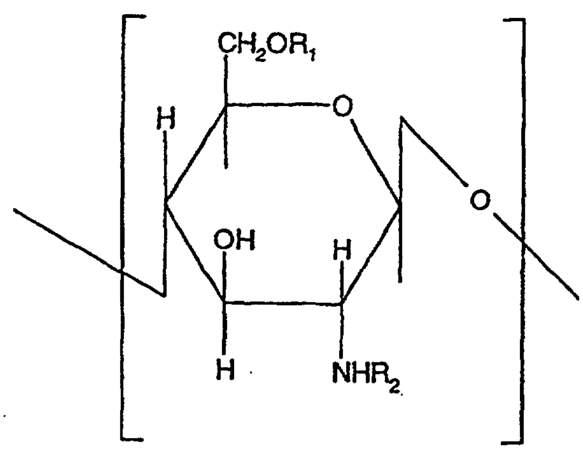

(I)

où
$R_1$ signifie H dans 0% à 82% du temps et carboxyméthyle dans 18%-100% du temps, et où $R_2$ signifie H dans 50% à 100% du temps et acétyle dans 0% à 50% du temps; et où le O en position 3 peut être substitué avec

$R_1$ quelquefois, mais l'espèce indiquée est l'espèce prédominante,
(b) 0,01 à 10 pourcents en poids d'un agent de libération ophtalmique; et
(c) 99,89 à 74,99 pourcents en poids d'un véhicule ophtalmique.

**2.** Une composition ophtalmique selon la revendication 1, où ladite composition ophtalmique a un pH de 4 ou plus.

**3.** Une composition ophtalmique selon la revendication 1, comprenant de 1 à 10 pourcents en poids d'O-carboxyméthyl-chitosan.

**4.** Une composition ophtalmique selon la revendication 1, comprenant :

(a) 1 à 6 pourcents en poids d'O-carboxyméthyl-chitosan;
(b) 0,01 à 2 pourcents en poids d'un agent de libération ophtalmique; et
(c) 98,99 à 93,99 pourcents en poids d'un véhicule ophtalmique.

**5.** Une composition ophtalmique selon la revendication 1, où ledit agent de libération se dégrade pendant le stockage dans des solutions à un pH qui n'est pas acide.

**6.** Une composition ophtalmique selon la revendication 1, où ledit agent de libération est choisi parmi le groupe comprenant la 3H-thymidine, le chlorure d'acétylcholine, l'acyclovir, l'adrénaline, l'améthocaïne, l'acide aminocaproïque, le phosphate d'antazoline, l'acide arachidonique, l'atropine, le chlorhydrate de benoxinate, le chlorhydrate de bétaxolol, la bupivacaïne, le carbachol, le cartéolol, le chloramphénicol, le chlorhydrate de chlortétracycline, la chymatrypsine, la clonidine, la cocaïne, la corynanthine, l'acide cromoglicique, le cyclopentolate, le bromure de démécarium, la dexaméthasone, la dibutoline, le dichlorphénamide, le diclofénac, le chlorhydrate de dipivéfrine, l'iodure d'échodtiophate, l'éphédrine, le bitartrate d'épinéphrine, l'érythromycine, l'éthambutol, l'étidocaïne, l'eucatropine, la fluorométhalone, la fluorométholone, le sulfate de gentamicine, la gramicidine, la H-thymidine, le méthylbromure d'homatropine, l'acide hyaluronique, l'hydrocortisone, l'idoxuridine, l'indométhacine, le triphosphate d'inositol, les phosphates d'inositol, l'isoflurophate, l'isosorbide, la lachésine, le lévobunolol, la lévocabastine, la lidocaïne, la lignocaïne, la médrysone, la mépivacaïne, la méthacholine, le méthazolamide, le chlorhydrate de naphazoline, la natamycine, le sulfate de néomycine, la néostigmine, la noradrénaline, l'ofloxacine, l'oxybuprocaïne, l'oxymétazoline, l'oxyphénonium, le maléate de phéniramine, le chlorhydrate de phényléphrine, les phosphates de phosphatidylinositol, la physostigmine, le chlorhydrate de pilocarpine, les polyhéxaméthylène-biguanides, les sulfates de polymyxine B, le phosphate sodique de prédnisolone, le chlorhydrate de proparacaïne, la proxyméthacaïne, le maléate de pyrilamine, le bromohydrate de scopolamine, le sorbinil, le sulfacétamide, la sulfisoxazoledisolamine, le tamoxifène, le chlorhydrate de tétracaïne, la tétracycline, le chlorhydrate de tétrahydrozoline, le maléate et l'hémihydrate de timolol, la trifluridine, le tropicamide, la vidarabine, leurs autres sels et leurs mélanges ophtalmiquement acceptables.

**7.** Une composition ophtalmique selon la revendication 1, où ledit agent de libération est choisi parmi le groupe comprenant la pilocarpine, l'épinéphrine, la dipivéfrine, l'hydralazine, le carbachol, leurs sels et leurs mélanges ophtalmiquement acceptables.

**8.** Une composition ophtalmique selon la revendication 7, où ledit agent de libération est l'hydralazine ou un de ses sels ophtalmiquement acceptables.

**9.** Une composition ophtalmique selon la revendication 1, où ladite composition a une viscosité de 50 à 500 mPas (centipoises) à 25°C.

**10.** Une composition ophtalmique selon la revendication 1, où ladite composition est un liquide lacrymal artificiel.

**11.** Une composition ophtalmique selon la revendication 1, où ladite composition a une viscosité de 50 à 100 000 mPas (centipoises) à 25°C.

**12.** Une composition ophtalmique selon la revendication 1, comprenant de 1 à 6 pourcents en poids d'O-carboxyméthyl-chitosan.

**13.** Une composition ophtalmique selon la revendication 1, comprenant:

(a) 1 à 6 pourcents en poids d'O-carboxyméthyl-chitosan;
(b) 0,01 à 2 pourcents en poids d'un agent de libération ophtalmique; et
(c) 98,99 à 93,99 pourcents en poids d'un véhicule ophtalmique.

**14.** Une composition ophtalmique selon la revendication 13, où ledit agent de libération est choisi parmi le groupe comprenant la pilocarpine, l'épinéphrine, la dipivéfrine, l'hydralazine, le carbachol, leurs sels et leurs mélanges ophtalmiquement acceptables; et où le pH de ladite composition ophtalmique est supérieur à 4.

**15.** Une composition ophtalmique selon la revendication 1, où ledit O-carboxyméthyl-chitosan a été placé dans un autoclave à des températures élevées de 100 à 150°C pendant un temps de 5 à 60 minutes.

**16.** Utilisation d'O-carboxyméthyl-chitosan de formule (I) selon la revendication 1, pour la préparation d'une composition ophtalmique selon la revendication 1, pour augmenter la rétention oculaire et/ou la biodisponibilité d'un agent de libération ophtalmique.

FIG. 1

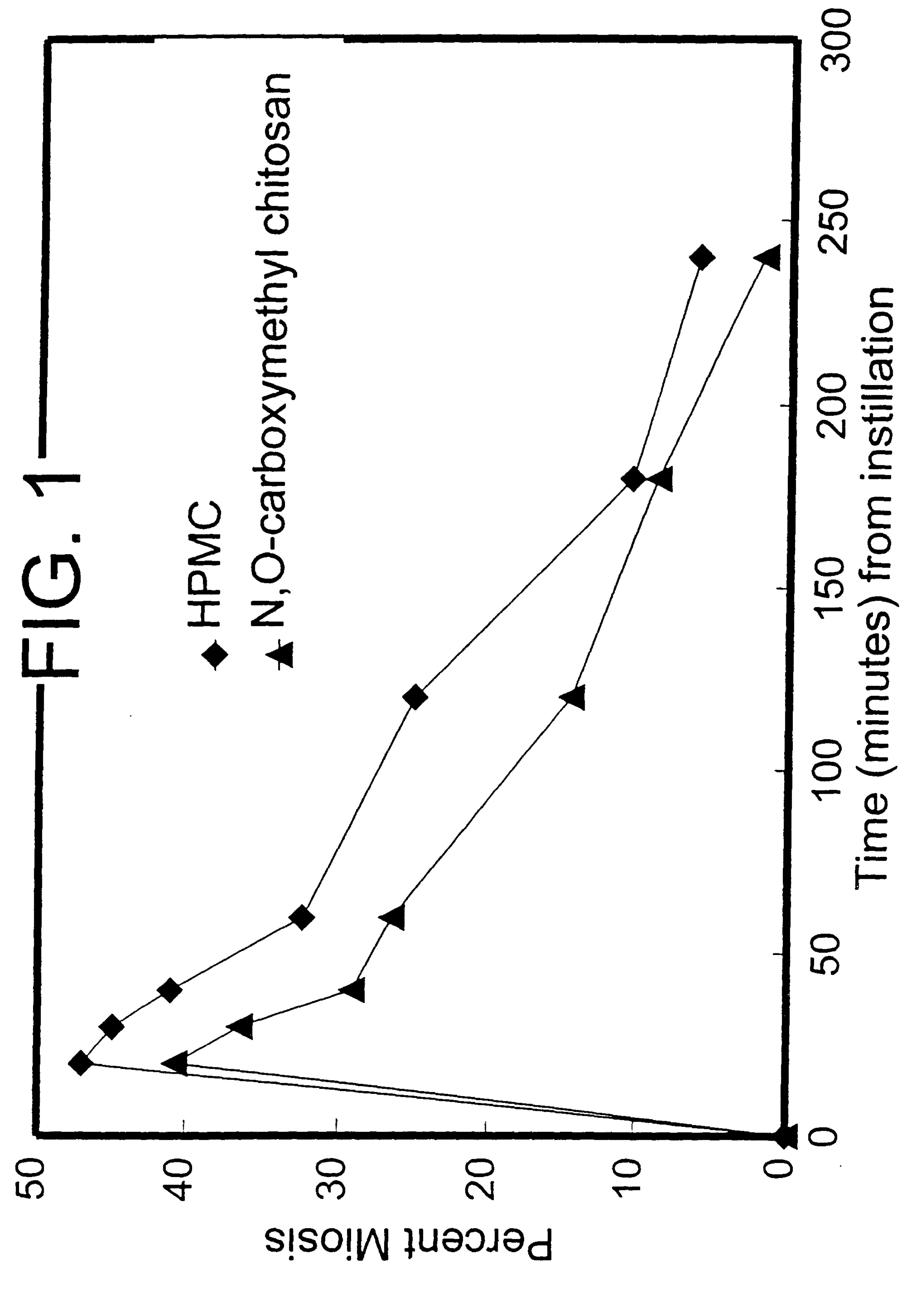
HPMC
N,O-carboxymethyl chitosan
Percent Miosis
0
10
20
30
40
50
Time (minutes) from instillation
0
50
100
150
200
250
300

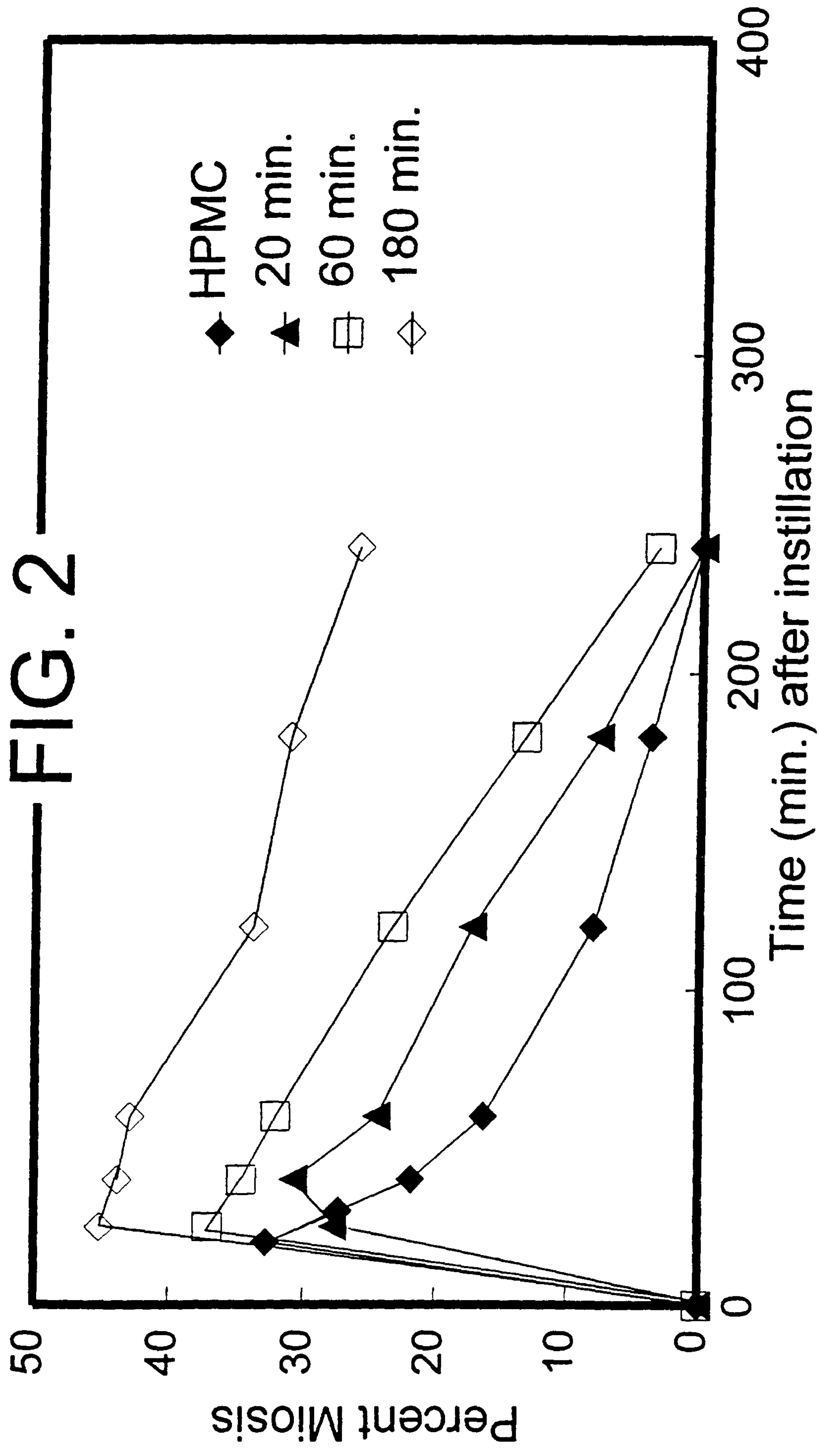
HPMC
20 min.
60 min.
180 min.
Percent Miosis
0
10
20
30
40
50
0
100
200
300
400
Time (min.) after instillation

FIG. 2

FIG. 3

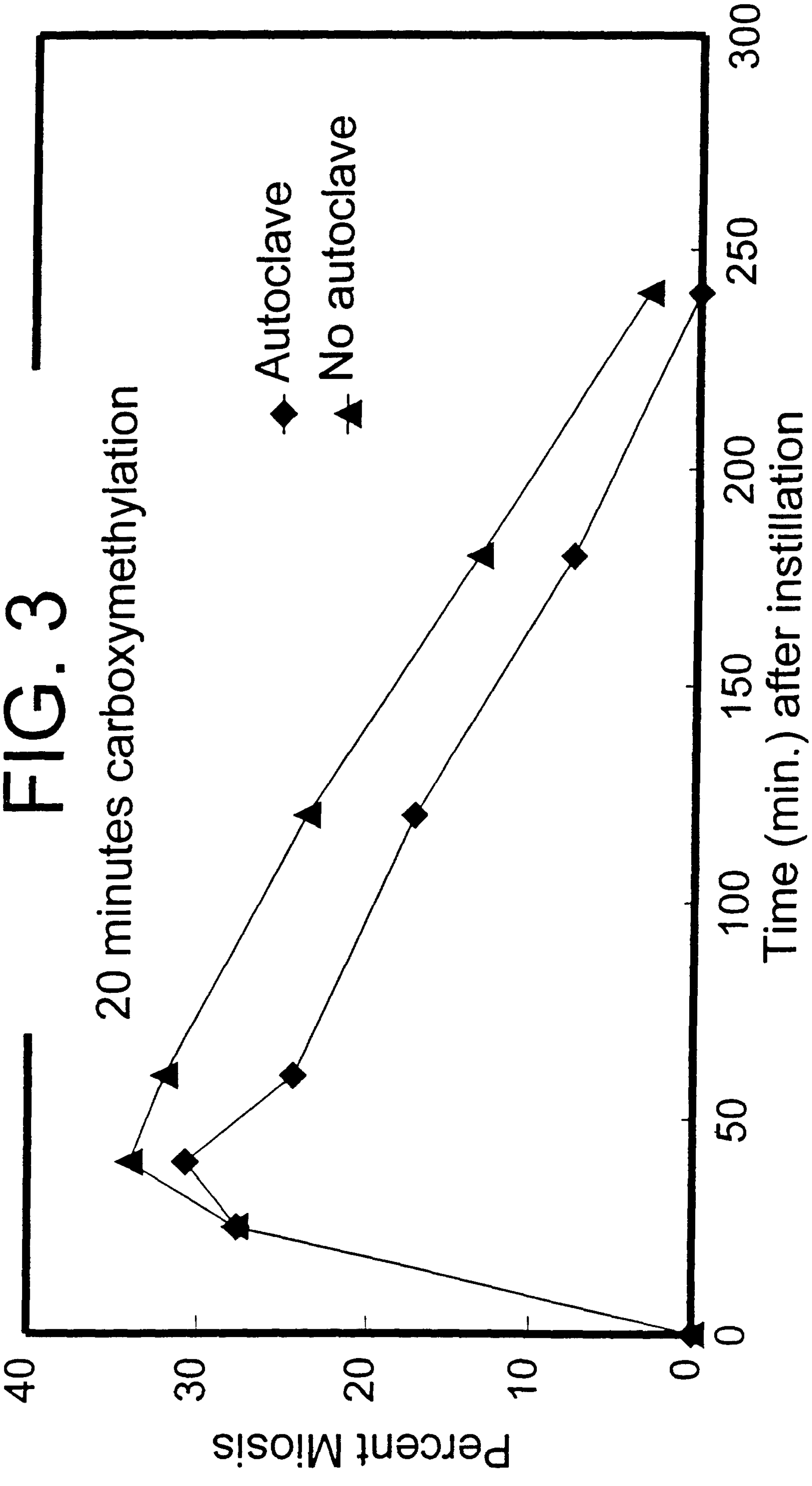
20 minutes carboxymethylation
Autoclave
No autoclave
Percent Miosis
0
10
20
30
40
0
50
100
150
200
250
300
Time (min.) after instillation

FIG. 4

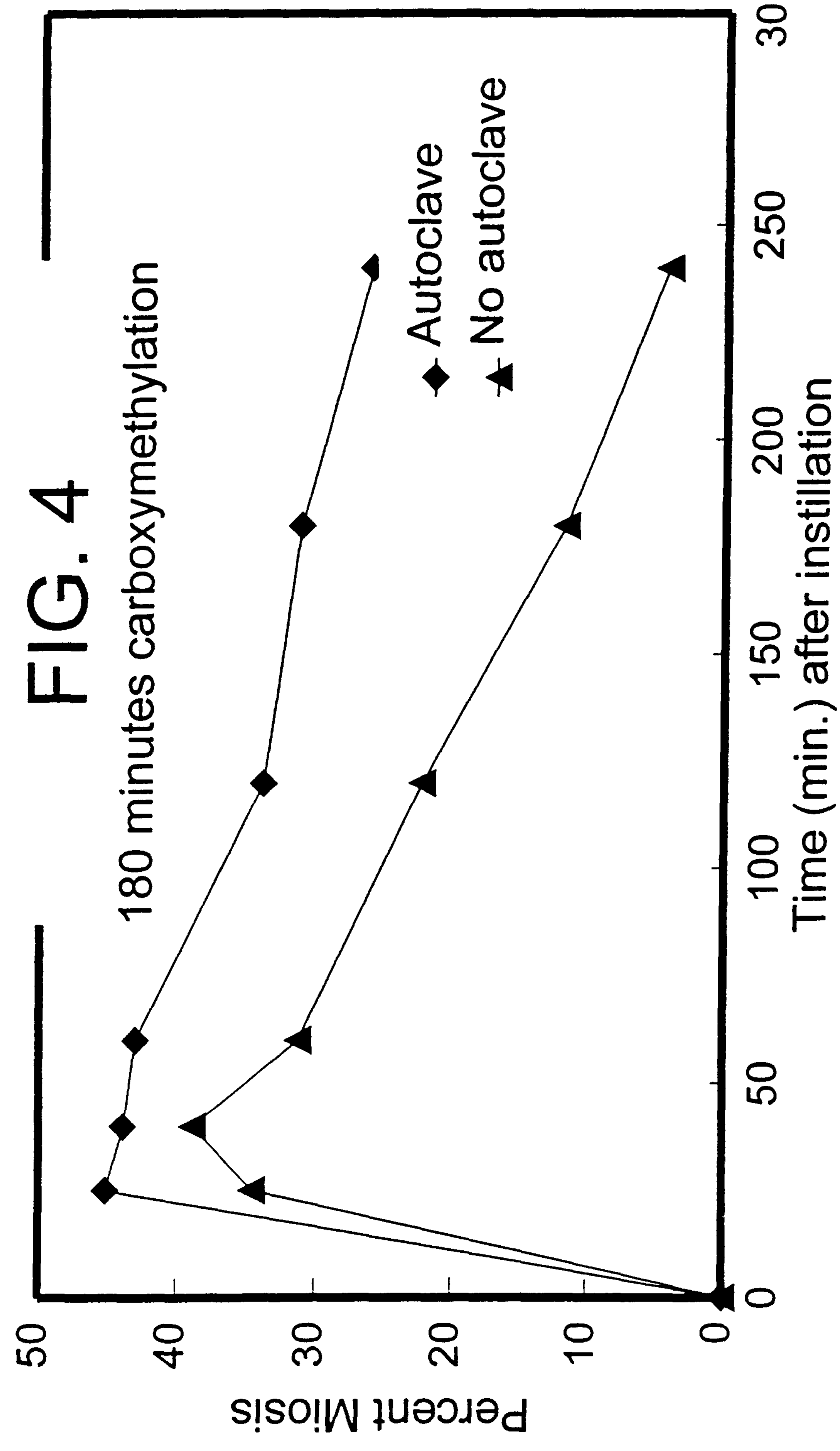
180 minutes carboxymethylation
Percent Miosis
0
10
20
30
40
50
Time (min.) after instillation
0
50
100
150
200
250
30
Autoclave
No autoclave